Europäisches Patentamt

(19) European Patent Office

Office européen des brevets

(11) Publication number: **0 019 478**
**A2**

## (12) EUROPEAN PATENT APPLICATION

(21) Application number: 80301623.7

(22) Date of filing: 19.05.80

(51) Int. Cl.³: **G 01 J 3/50**
**A 61 B 5/14**

(30) Priority: 19.05.79 GB 7917486

(43) Date of publication of application:
26.11.80 Bulletin 80/24

(84) Designated Contracting States:
AT BE CH DE FR GB IT LI LU NL SE

(71) Applicant: FIFE REGIONAL COUNCIL
Fife House North Street
Glenrothes, Fife KY7 5LT(GB)

(72) Inventor: Reilly, William Joseph
7, Murray Place
St. Andrews, Fife, Scotland(GB)

(74) Representative: Wotherspoon, Graham et al,
FITZPATRICKS 48 St. Vincent Street
Glasgow G2 5TT(GB)

(54) Apparatus and method for indicating a colour change of an object.

(57) An incident light beam from a light source (7) is guided by a light guide (11) onto the skin surface (5). Infra-red radiation is removed from the reflected beam by a filter 20 located between the skin surface and two phototransistors (14, 15). The phototransistors (14, 15) detect the reflected light and has red and blue filters respectively. The outputs of the phototransistors are compared in a differential amplifier (27) and the differential amplifier output signal (29) is adjusted to zero by an offset bias circuit (28) for no colour change. When the surface changes colour the red and blue optical filters pass different amount of light and a difference is produced between the phototransistor outputs. This difference is amplified by the differential amplifier to provide an output signal representative of the colour change of the surface.

Fig.2.

EP 0 019 478 A2

Croydon Printing Company Ltd.

Apparatus and Method for Indicating the Colour
Change of an Object

The present invention relates to apparatus for indicating the colour change of a surface.

As is known, a light detector can be made sensitive to a particular colour by the insertion of an appropriate filter between the light source and the detector. If, when the change of colour of a surface is accompanied by a change in the surface thermal emissivity, which can occur, for example, in skin when blood flow increases due to blushing, the skin reflects incident light to indicate colour which can be detected photoelectrically. If incident light is provided by an incandescent light source e.g. the sun or a tungsten filament lamp, much of radiation will be in the infra-red region. Devices like phototransistors tend to be more sensitive to infra-red radiation than visible radiation and therefore reflected infra-red radiation from the skin will swamp small changes in the visible radiation due to slight changes in skin colour. This results in the apparatus being insensitive to small variations in colour.

In addition, many colour changes occur at irregular intervals, such as the ripening of fruit, and it is desirable to know when the fruit is about to ripen so that it can be picked. Conventional colour detecting apparatus may provide an indication of the colour of the fruit but does not relate this to any previous colour, so accurate prediction of ripening, which may be indicated by a particular colour change is difficult.

An object of the present invention is to obviate or mitigate the abovesaid disadvantages.

According to the present invention there is provided apparatus for indicating a colour change of a surface comprising, an incident light source for illuminating the surface, at least one light detector for detecting light reflected from said surface, means for providing a reference colour signal, and means for comparing the light reflected from the surface with the reference colour signal, whereby when the surface changes colour, a signal indicative of said colour change is produced.

According to the present invention there is provided a method of indicating a colour change of a surface comprising:-

illuminating the surface with incident light,

detecting light reflected from the surface which is indicative of the colour of the surface,

adjusting the detected light signal to give a reference signal,

continuously comparing the detected light reflected with the reference signal to provide an output signal indicative of a surface colour change.

In particular, it is envisaged that the apparatus and method according to the present invention will be suited to any application where it is desired to measure a change in colour; for example, in skin where the colour of the skin may change due to blushing, drug action, bruising, blood restriction and radiation of all kinds.

A first embodiment of the present invention will be described by way of example with reference to the accompanying drawings in which:-

Fig. 1 is a view of part of the apparatus according to the present invention;

Fig. 2 is a view of Fig. 1 taken along the direction A - A;

Fig. 3 is a view of part of Fig. 1;

Fig. 4 is a view of Fig. 3 taken along the direction B - B;

Fig. 5 is a signal processing circuit according to the present invention; and

Fig. 6 is an alternative processing circuit according to the present invention.

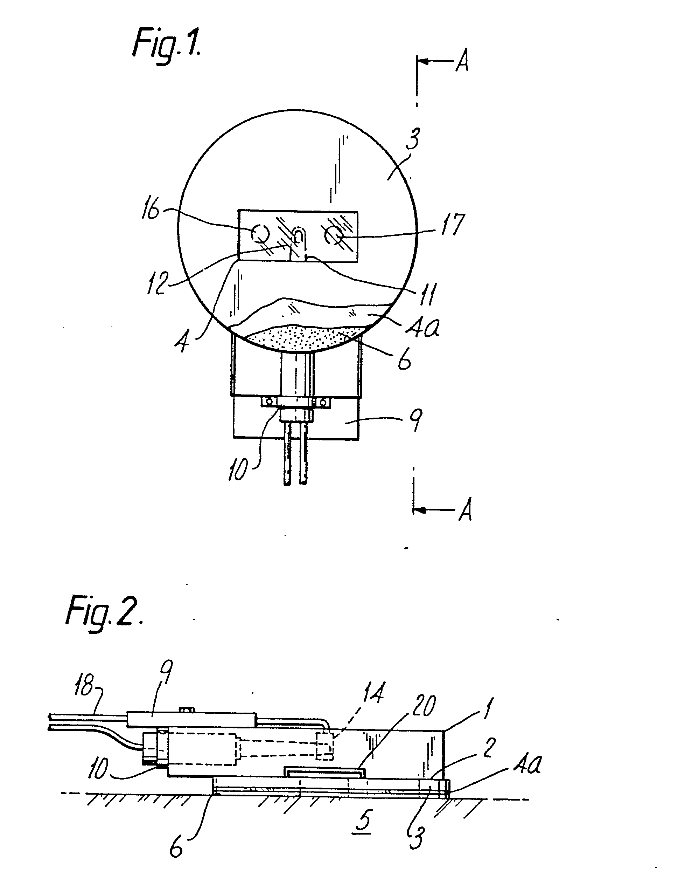

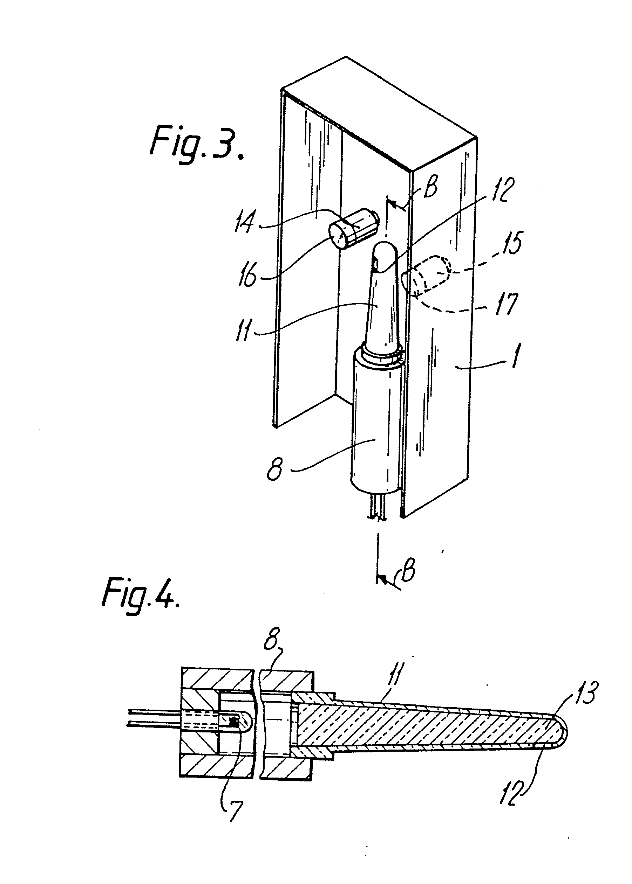

Referring now to Figs. 2 and 3 of the drawings a colour change indicator sensor has a housing 1 having a flat surface 2 on which flat circular plate 3 is mounted. The plate 3 has a rectangular aperture 4. The rectangular aperture 4 is covered with a transparent material 4a. The plate 3 is securable to the skin surface 5 by a ring-shaped double-sided adhesive tape 6. A light source in the form of a lamp 7 is contained in a lamp housing 8 (Fig. 4) which is secured to a plate 9 by a clamp 10. The plate 9 is secured to the housing 1.

Referring now to Figs. 3 and 4 the lamp housing 8 has a light guide 11 having a window 12 located therein, and the guide 11 contains an infra-red filter 13 which is conveniently perspex. The lamp housing 8 is clamped to the housing 1 such that the window 12 is opposite the aperture 12 (Fig. 1). Two phototransistors 14, 15 are mounted on the housing 1 opposite the aperture 4 in the plate 3 such that the window 12 of the guide 11 is located between said phototransistors 14, 15 on the same axis, as shown in Fig. 2. Phototransistors 14, 15 have filters 16, 17 mounted thereon respectively and are connected by conductors to a cable 18 which is secured to the housing 1 by a clamp 19. An infra-red filter 20 is located between the aperture 4 and the phototransistors 14, 15 (Fig. 2).

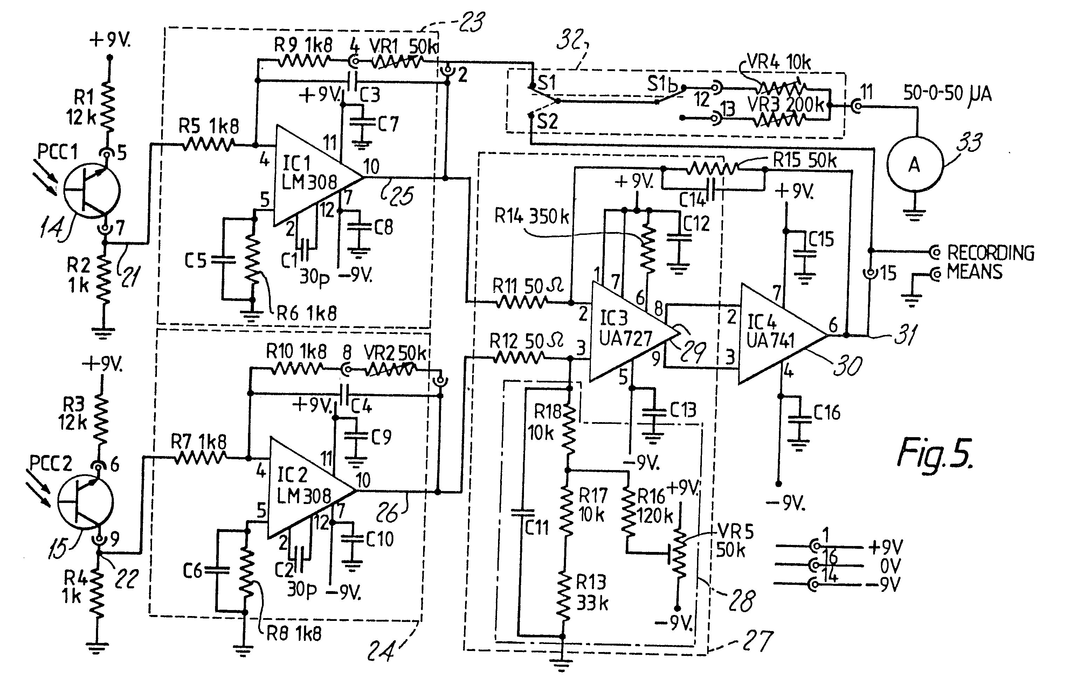

Referring now to Fig. 5 phototransistors 14, 15 have output terminals 21, 22 which are connected to the noninverting inputs of integrated circuit amplifiers 23 and 24 respectively. Each amplifier has respective outputs 25, 26 which are connected to a differential amplifier 27. The differential amplifier 27 is internally compensated for variations in operating, or ambient, temperature. The differential amplifier 27 includes an offset bias circuit 28 which enables the differential amplifier output 29 to be adjusted to zero when there is no input or when both input signals are equal. The output 29 of the differential amplifier is connected to an output driver 30 which has an output 31 connected to an indicating or recording means (not shown) for displaying a signal indicative of the surface colour, or change of colour. The output of the amplifier 23 and the output of the output driver 29 are connected via a switching and sensitivity adjustment means 32 to an ammeter 33. The switching means 32 has two switch positions; in position S1 the amplifier 23 is connected to the ammeter 33 and in position S2 the output driver 29 is connected to the ammeter 33.

In use, the plate 3 is located on the appropriate skin site by the tape 6 and the apparatus is switched on. The lamp 7 illuminates and incident radiation is guided from the window 12 through the aperture 4 and transparent covering material 4a onto the surface of the skin 5. The light reflected from the skin surface 5 enters the aperture 4, via transparent material 4a and is filtered by the infra-red filter 20 to exclude infra-red radiation. The filtered reflected radiation is received by the phototransistors 14, 15 via red and blue filters 16, 17 respectively. The red and blue filters have different optical transmissivities, therefore the amount of incident radiation detected, and hence the respective output signals produced by the phototransistors are different, the output of phototransistor 14 being greater than the output from phototransistor 15. This difference is amplified by the differential amplifier 26 and appears at the output of the output driver 30. In order to set the initial conditions, that is, to set the output 30 to read zero when there is no surface colour change displayed on the ammeter 33, the switch 32 is switched to position S2 and the offset bias circuit 27 is adjusted until the ammeter 33 reads zero. The initial operating condition of the colour change indicator is therefore set i.e. there is zero output for no colour change.

Once the circuit output is initially set to zero for zero colour change the colour change indicator is fully

- 5 -

operational. The switch 32 is switched to position S1 and a signal indicative of the surface colour is continuously displayed on the ammeter 33 via the phototransistor 14 and the amplifier 23. This surface colour signal is used as a reference signal to indicate that the illuminating, detecting and signal processing apparatus are functioning properly.

When the subject is under stress, either physical or psychological, blood flow to the skin surface increases, particularly on the face, and the skin colour changes to pinky-red which is characteristic of blushing. The optical components of the reflected light change so that the ratio of red to blue light is increased, consequently, phototransistor 14 receives more light than phototransistor 15, and a difference between electrical outputs 20, 21 is produced. This difference is amplified by the differential amplifier 26 and the output driver 27 and a d.c. shift in output 31 of the output driver 30 is produced which is indicative of a colour change. The output signal can be displayed or recorded on a chart recorder to provide a permanent record of colour changes.

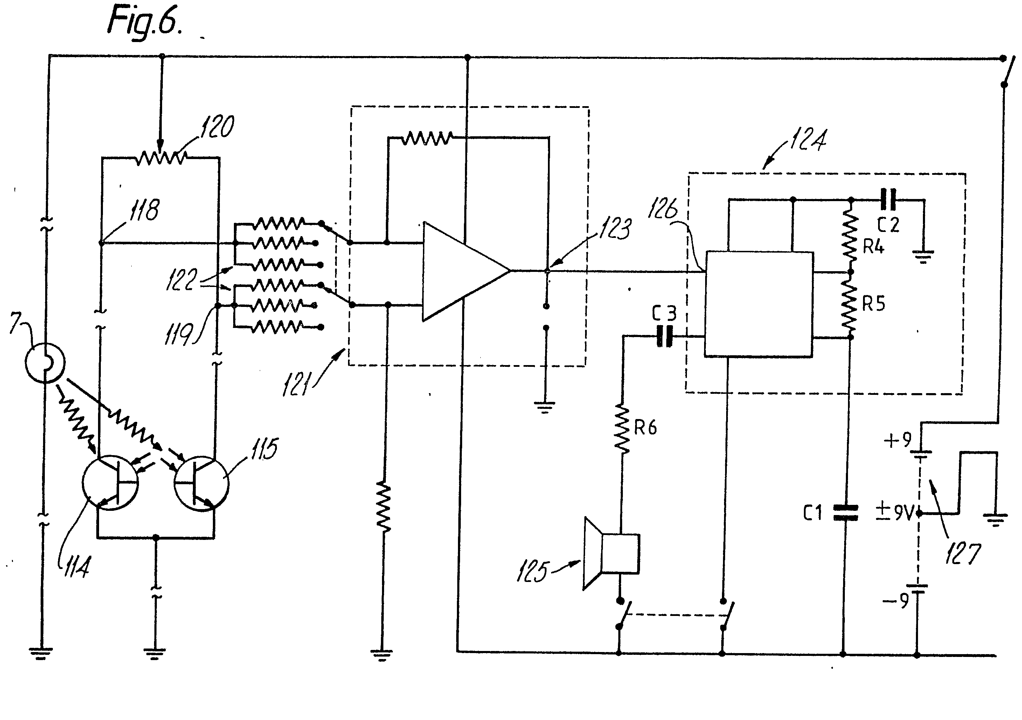

Without departing from the scope of the invention several modifications may be made to the apparatus described in the embodiment. For example, an alternative signal processing circuit (Fig. 6) can be used to indicate a colour change. The phototransistors 114, 115 have respective outputs 118, and 119 which are connected via a potentiometer 120. The outputs 118, 119 are also connected to the respective inputs of a differential amplifier 121 via 3-way rotary gain switches 122. The differential amplifier output 123 is connected to a voltage controlled oscillator 124 which has a base frequency dependent on the selected values of R4, R5 and C1. The oscillator output drives a loudspeaker 125 via an output link, R6 and C3 in series. The values of R4, R5 and C6 are selected so that the 'lo' period is very short when compared to the 'hi' period, which permits the oscillator frequency to be varied over a wide range by modulating the input to the control voltage terminal 126. The capacitor C2

decouples the oscillator 124 from electrical noise on the earth line. Power is supplied to the components of the current by a 9v. d.c. supply 127.

In operation, using the previously referred to sensor (Figs. 1 - 4) the plate 3 is located on the appropriate skin site by the tape 6 and the apparatus is switched on. The lamp 7 illuminates and the incident light is guided from the window 12 through the aperture 4 onto the surface of the skin 5. The incident light is reflected from the skin surface 5 and is received by phototransistors 114, 115 via filters 116, 117 which are red and blue coloured respectively. The filters 116, 117 have different optical transmissivities and the difference between the outputs 118, 119 of the phototransistors 114, 115 is minimised by adjustment of the potentiometer 120 so that the output of the loudspeaker 125 is set at a particular frequency for the ambient skin colour.

When a skin colour change occurs, difference is amplified by the differential amplifier 121 and the resulting amplifier output signal 123 modulates the oscillator frequency thereby changing the pitch of the signal produced by the loudspeaker 125 and providing an audible indication of the skin colour change due to blushing or other.

Furthermore, without departing from the scope of the invention the following arrangements provide an indication of the colour change in only one colour.

The colour change indicator sensor may have an aperture positioned in the plate 3 so that only one phototransistor receives light reflected from the skin. The other phototransistor being located opposite a constant colour whereby the output of the phototransistor is constant and acts as a reference level for the output of the phototransistor which receives reflected light.

A reference level may also be provided by replacing a phototransistor by a constant voltage source against which the reflected light signal can be continously compared.

In addition, the output from one detector can be used as negative feedback reference when natural changes in skin colour occur, while indicated changes due to drug action for example, are measured with the other detector.

The sensor shown in Figs. 1 - 2 may be also located at a fixed distance from the surface and, in the case of a compliant surface, the transparent material 4a, which prevents identation of the surface by the aperture 4, is not required.

Advantages of the embodiment and modifications thereof are that the apparatus may be connected directly to a chart recorder to provide a permanent record of surface colour; the apparatus can be used for biofeedback experiments ,whereby subjects can teach themselves to control blushing for example, until no audible pitch change or visual display shift is detectable. The apparatus provides a reliable indication of colour change of a surface against a constantly varying background colour. A 9v. d.c. supply is used which provides minimum hazard against electrical injury to the subject in a fault situation, and since the plate 3 is securely attached to the skin surface 5, movements have a minimal effect on the signal obtained due to a colour change.

CLAIMS:

1. Apparatus for indicating the change in colour of a surface, comprising an incident light source for illuminating the surface, at least one light detector for detecting light reflected from the surface, means for providing a reference signal, and means for comparing the light reflected from the surface with the reference signal whereby when the surface changes colour a signal indicative of said colour change is produced.

2. Apparatus as claimed in claim 1 wherein said means for providing a reference signal is a constant voltage source.

3. Apparatus as claimed in claim 1 wherein said means for providing a reference signal is a light detector, the apparatus having two detectors.

4. Apparatus as claimed in claim 2 wherein the detector has a light filter for filtering reflected light.

5. Apparatus as claimed in claim 3 where one or both detectors has a light filter for filtering reflected light.

6. Apparatus as claimed in claim 5 wherein one detector has a red filter, the other detector having a blue filter.

7. Apparatus as claimed in any preceding claim wherein an infra-red light filter is located between the surface and the or each detector.

8. Apparatus as claimed in any preceding claim wherein an infra-red filter is located between the light source and the surface.

9. Apparatus as claimed in any preceding claim wherein the means for comparing the light reflected from the surface with the reference signal is a differential amplifier circuit.

10. Apparatus as claimed in claim 9 wherein the signal indicative of a colour change is a modulated frequency signal in the audible range and is preferably connected to a loudspeaker.

11. Apparatus as claimed in claim 9 wherein the signal indicative of a colour change is a varying direct current (d.c.) shift.

12. A method of indicating a colour change of a surface comprising, illuminating the surface with incident light, detecting light reflected from the surface which is indicative of the colour of the surface, adjusting the detected light signal to give a reference signal, continuously comparing the detected light reflected with the reference signal to provide an output signal indicative of a surface colour change.

Fig.1.

Fig.2.

Fig. 3.

Fig. 4.

Fig.5.

Fig.6.